# EUROPÄISCHE PATENTANMELDUNG

(11) **EP 1 959 019 A1**
(43) Veröffentlichungstag der Anmeldung: **20.08.2008**
(21) Anmeldenummer: 06126993.2
(22) Anmeldetag: 22.12.2006
(51) Int. Cl.: C12P 7/24, C12P 7/26, C12P 7/46, C12P 41/00

(54) **Verfahren zur enzymatischen Reduktion von Alkenderivaten**

(71) Anmelder: BASF SE, 67056 Ludwigshafen (DE)
(72) Erfinder: Die Erfindernennung liegt noch nicht vor

(57) **Zusammenfassung**

Verfahren zur enzymatischen Herstellung von Verbindungen der allgemeinen Formel (2)
aus ungesättigten Alken-Derivaten der allgemeinen Formel (1) durch Reduktion einer Verbindung der Formel (1) in Gegenwart einer Reduktase umfassend wenigstens eine der Polypeptidsequenzen SEQ ID NO:1, 2, oder 3 oder mit einer funktional äquivalenten Polypeptidsequenz, die mindestens 80% Sequenzidentität mit SEQ ID NO:1, 2, oder 3 aufweist.

## Beschreibung

Die Erfindung betrifft ein Verfahren zur enzymatischen Reduktion von Alkenderivatender allgemeinen Formel (1).

### Aufgabenstellung

Es bestand die Aufgabe, ein Verfahren zur enzymatischen Herstellung von Verbindungen der allgemeinen Formel (2) aus ungesättigten Alken-Derivaten der allgemeinen Formel (1) bereitzustellen, das insbesondere in hoher chemischer Ausbeute und sehr guter Stereoselektivität abläuft.

### Kurzfassung der Erfindung

Obige Aufgabe wurde gelöst durch Verwendung der Reduktasen YqjM, OPR1, OPR3 und funktionaler Äquivalente davon zur Reduktion von Alkenderivaten der allgemeinen Formel (1).

### Detaillierte Beschreibung der Erfindung

Die vorliegende Erfindung betrifft ein Verfahren zur enzymatischen Herstellung von Verbindungen der allgemeinen Formel (2) aus ungesättigten Alken-Derivaten der allgemeinen Formel (1) 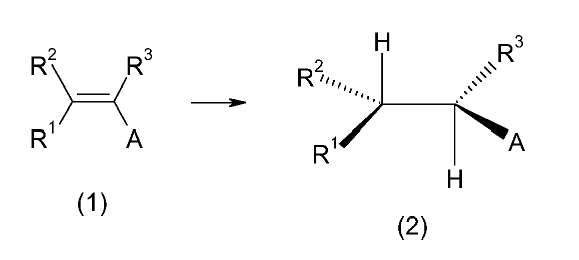 worin
A für einen Iminrest (=NR), einen Nitrorest (-NO₂), einen Ketonrest (-CRO), einen Aldehydrest (-CHO) einen Carboxylrest (-COOR) mit R= H oder gegebenenfalls substituerter C₁-C₆- Alkylrest, steht,
R¹ , R² und R³ unabhängig voneinander für H, C₁-C₆- Alkyl, C₂-C₆- Alkenyl, Carboxyl oder einen gegebenenfalls substituierten carbo- oder heterocyclischen, aromatischen oder nichtaromatischen Rest steht, oder R¹ ist mit R³ so verknüpt, dass sie Teil eines 4-8 gliedrigen Cyclus werden, oder R¹ ist mit R so verknüpt, dass sie Teil eines 4-8 gliedrigen Cyclus werden,
   durch Reduktion einer Verbindung der Formel (1) in Gegenwart einer Reduktase
   (i) umfassend wenigstens eine der Polypeptidsequenzen SEQ ID NO:1, 2, oder 3 oder
   (ii) mit einer funktional äquivalenten Polypeptidsequenz, die mindestens 80% Sequenzidentität mit SEQ ID NO:1, 2, oder 3 aufweist.

Grundsätzlich ist das erfindungsgemäße Verfahren sowohl mit gereinigten oder angereicherten Enzym selbst als auch mit Mikroorganismen, welche dieses Enzym natürlich oder rekombinant exprimieren, oder mit davon abgeleiteten Zellhomogenaten durchführbar.

Werden keine anderen Angaben gemacht, so bedeutet:
- C₁-C₆-Alkyl insbesondere Methyl, Ethyl, Propyl, Butyl, Pentyl oder Hexyl, sowie die entsprechenden ein- oder mehrfach verzweigte Analoga, wie i-Propyl, i-Butyl, sec.-Butyl, tert.-Butyl, i-Pentyl oder Neopentyl; wobei insbesondere die genannten C₁-C₄-Alkylreste bevorzugt sind;
- C₂-C₆-Alkenyl insbesondere die einfach ungesättigten Analoga der oben genannten Alkylreste mit 2 bis 6 Kohlenstoffatomen, wobei insbesondere die entsprechenden C₂-C₄-Alkenylreste bevorzugt sind,
- Carboxyl insbesonders die Gruppe COOH,
- Carbo- und heterocyclische aromatische oder nichtaromatische Ringe insbesondere gegebenenfalls kondensierte Ringe mit 3 bis 12 Kohlenstoffatomen und gegebenenfalls 1 bis 4 Heteroatomen, wie N, S und O, insbesondere N oder O. Als Beispiele können genannt werden Cyclopropyl, Cyclobutyl, Cyclopenty, Cyclohexyl, Cycloheptyl, die ein- oder mehrfach ungesättigten Analoga davon, wie Cyclobutenyl, Cyclopentenyl, Cyclohexenyl, Cycloheptenyl, Cyclohexadienyl, Cycloheptadienyl; Phenyl und Naphthyl; sowie 5- bis 7-gliedrige gesättigte oder ungesättigte heterocyclische Reste mit 1 bis 4 Heteroatomen, die ausgewählt sind unter O, N und S, wobei der Heterocyclus gegebenenfalls mit einem weitern Heterocyclus oder Carbocyclus kondensiert sein kann. Insbesondere sind zu nennen heterocyclische Reste abgeleitet von Pyrrolidin, Tetrahydrofuran, Piperidin, Morpholin, Pyrrol, Furan, Thiophen, Pyrazol, Imidazol, Oxazol, Thiazol, Pyridin, Pyran, Pyrimidin, Pyridazin, Pyrazin, Cumaron, Indol und Chinolin. Gegebenenfalls können die cyclischen Reste, aber auch die oben genannten Alkyl- und Alkenylreste ein- oder mehrfach, wie z. B. 1-, 2- oder 3-fach, substituiert sein. Als Beispiel für geeignete Substituierte sind zu nennen: Halogen, insbesondere F, Cl, Br; - OH, -SH, -NO₂, -NH₃, -SO₃H, C₁-C₄-Alkyl und C₂-C₄-Alkenyl, C₁-C₄ -Alkoxy; und Hydro- xy-C₁-C₄-Alkyl; wobei die Alkyl- und Alkenyl-Reste wie oben definiert sind und die Alkoxy-Reste von den oben definierten korrespondierenden Alkylresten abgeleitet sind.

Die Reste R¹ und R³ können auch so direkt miteinander verknüpft sein, dass sie zusammen mit der zu reduzierenden Doppelbindung einen 4-8, bevorzugt einen 5 oder 6 gliedrigen Cyclus bilden, beispielsweise eine Cyclopenten oder Cyclohexenstruktur, die auch gegebenenfalls substituiert sein kann, beispielsweise durch Alkyl-, bevorzugt Methylreste.

Die Reste R¹ und R können auch so direkt miteinander verknüpft sein, dass sie zusammen mit der zu reduzierenden Doppelbindung einen 4-8, bevorzugt einen 5 oder 6 gliedrigen Cyclus bilden, beispielsweise eine Cyclopenten oder Cyclohexenstruktur, die auch gegebenenfalls substituiert sein kann, beispielsweise durch Alkyl-, bevorzugt Methylreste.

Die o.g. 4-8 gliedrigen Cyclen können sowohl Carbocyclen sein, d.h. nur C-Atome bilden den Cyclus, als auch Heterozyklen, d.h. Heteroatome wie O;S;N, sind im Cyclus enthalten. Gewünschtenfalls können diese Carbo- oder Heterozyklen auch noch substituiert sein, d.h. H-Atome sind durch Heteroatome ersetzt. Beispielsweise sind N-Phenylsuccinimide (siehe unten Substrat 3) als solche substituierte Heterozyklen aufzufassen, die durch Cyclusbildung zwischen R¹ und R entstehen.

Besonders vorteilhafte Ausführungsformen der Erfindung sind die enzymatische Reduktion der folgenden Substrate (Verbindungen der allgemeinen Formel 1) zu den entsprechenden Verbindungen der allgemeinen Formel (2):

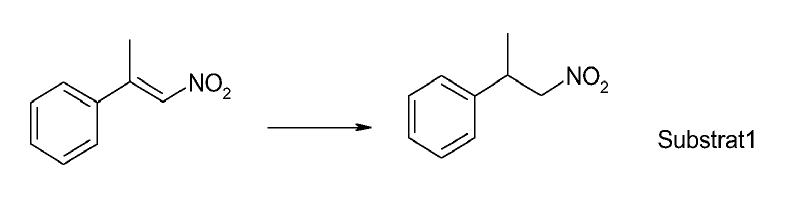

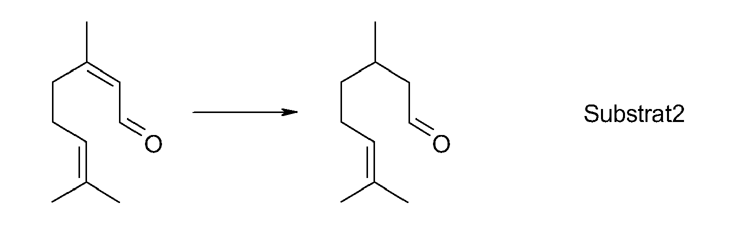

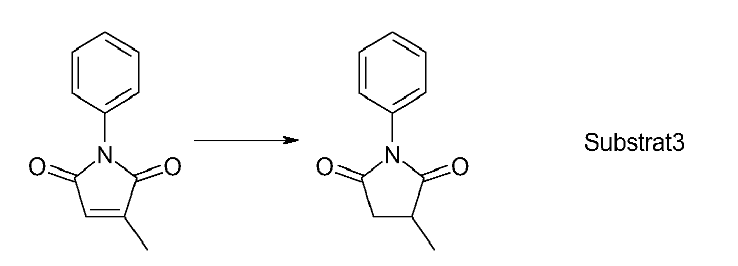

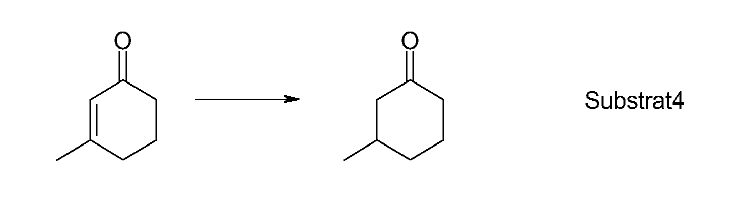

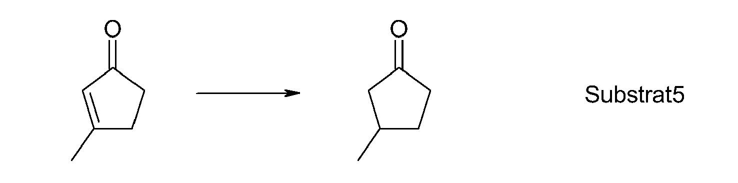

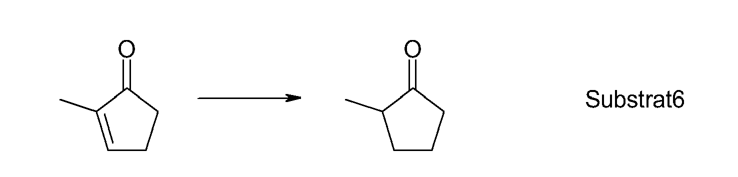

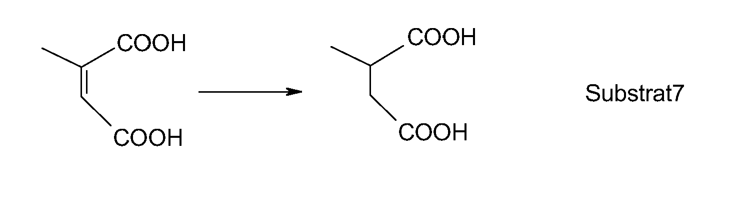

**Tabelle 1**

| Subtsrat | Cofactor | OPR1 | | OPR3 wt | | YqjM | |
|---|---|---|---|---|---|---|---|
| | | % | E.e. % | % | E.e. % | % | E.e. % |
| 1 | NADH | >99 | (*R*) 97 | 69 | (*S*) 82 | 94 | (*S*) 92 |
| 1 | NADPH | >99 | (*R*) 96 | 72 | (*S*) 87 | 85 | (*S*) 70 |
| 1 | NAD⁺-FDH | >90 | (*R*) 95 | 40 | (*S*) 75 | 50 | (*S*) 85 |
| 1 | NADP⁺-G6PDH | 90 | (*R*) 98 | 75 | (*S*) 93 | 47 | (*S*) 84 |
| 2 | NADH | >99 | (*S*)>95 | 90 | (*S*)>95 | 70 | (*S*)>95 |
| 2 | NADPH | >99 | (*S*)>95 | 90 | (*S*)>95 | 73 | (*S*)>95 |
| 2 | NAD⁺-FDH | Main product: Citronellol (saturated alcohol) | | | | | |
| 2 | NAD⁺-GDH | 20 | (*S*)>95 | 90 | (*S*)>95 | 3 | (*S*)>95 |
| 2 | NADP⁺-G6PDH | 15 | (*S*)>95 | 95 | (*S*)>95 | 57 | (*S*)>95 |
| 3 | NADH | 58 | (*S*) 61 | 27 | (*S*) 45 | 50 | (*S*) 55 |
| 3 | NADPH | 45 | (*S*) 64 | 19 | (*S*) 45 | 100 | (*S*) 66 |
| 3 | NAD⁺-FDH | 88 | (*R*) 1 | 65 | (*R*) 1 | 100 | (*R*) 2 |
| 3 | NADP⁺-G6PDH | 14 | (*S*) 61 | 10 | (*S*) 58 | 72 | (*S*) 94 |
| 4 | NADH | n.c. | - | 1 | (*S*)>99 | 0.5 | (*S*) 59 |
| 4 | NADPH | n.c. | - | 2 | (*S*)>99 | n.c. | - |
| 4 | NAD⁺-FDH | - | - | 5 | (*S*)>99 | 1 | (*S*) 64 |
| 4 | NADP⁺-G6PDH | - | - | 1 | (*S*)>99 | - | - |
| 5 | NADH | n.c. | - | 3 | (*S*)>99 | n.c. | - |
| 5 | NADPH | n.c. | - | 2 | (*S*)>99 | n.c. | - |
| 5 | NAD⁺-FDH | - | - | 11 | (*S*)>99 | - | - |
| 5 | NADP⁺-G6PDH | - | - | 1 | (*S*)>99 | - | - |
| 6 | NADH | 99 | (*R*)>99 | 99 | (*R*)>99 | 99.5 | (*R*)>99 |
| 6 | NADPH | 99 | (*R*)>99 | 99 | (*R*)>99 | 98.5 | (*R*)>99 |
| 6 | NAD⁺-FDH | 99 | (*R*) 97 | 99 | (*R*) 92 | 99 | (*R*) 92 |
| 6 | NADP⁺-G6PDH | 99 | (*R*) 96 | 99 | (*R*) 97 | 99 | (*R*) 96 |
| 7 | NADH | >99 | (*R*)>99 | n.c. | - | | |
| 7 | NADPH | >99 | (*R*)>99 | n.c. | - | | |
| 7 | NAD⁺ -FDH | 2 | (*R*)>99 | n.d. | - | | |
| 7 | NADP⁺ -G6PDH | 96 | (*R*)>99 | n.d. | - | | |

Das erfindungsgemäße Verfahren kann insbesondere mit Verbindungen der allgemeinen Formel (1) durchgeführt werden, in denen A für einen Aldehyd oder Ketonrest steht und R¹ oder R2 für Methyl steht.

Die für das erfindungsgemäße Verfahren geeigneten Reduktasen (welche zuweilen auch als Enoat-Reduktasen bezeichnet werden) besitzen eine Polypeptidsequenz gemäss SEQ ID NO:1, 2, oder 3 oder eine Polypeptidsequenz, die mindestens 80%, wie z.B. mindestens 90%, oder mindestens 95% und insbesondere mindestens 97%, 98% oder 99% Sequenzidentität mit SEQ ID NO: 1, 2 oder 3 aufweist.

Ein Polypeptid mit der SEQ ID NO:1 ist bekannt unter der Bezeichnung YqjM aus Bacillus subtilis. (UniprotKB/Swissprot entry P54550)

Ein Polypeptid mit der SEQ ID NO:2 wird kodiert vom OPR1 Gen aus Tomate. (UniprotKB/Swissprot entry Q9XG54)

Ein Polypeptid mit der SEQ ID NO:3 wird kodiert vom OYPR3 Gen aus Tomate.. (UniprotKB/Swissprot entry Q9FEW9).

Die Ermittlung der Sequenzidentität soll für die hier beschriebenen Zwecke durch das Computerprogramm "GAP" der Genetics Computer Group (GCG) der University of Wisconsin erfolgen, wobei die Version 10.3 unter Verwendung der von GCG empfohlenen Standardparameter zum Einsatz kommen soll.

Solche Reduktasen können ausgehend von SEQ ID NO: 1, 2, oder 3 durch dem Fachmann bekannte gezielte oder randomisierte Mutageneseverfahren erhalten werden. Alternativ kann jedoch auch in Mikroorganismen , bevorzugt in solchen der Gattungen *Alishewanella, Alterococcus, Aquamonas, Aranicola, Arsenophonus, Azotivirga, Brenneria, Buchnera (aphid P-endosymbionts), Budvicia, Buttiauxella, Candidatus Phlomobacter, Cedecea, Citrobacter, Dickeya, Edwardsiella, Enterobacter, Erwinia, Escherichia, Ewingella, Grimontella, Hafnia, Klebsiella, Kluyvera, Leclercia, Leminorella, Moellerella, Morganella, Obesumbacterium, Pantoea, Pectobacterium, Photorhabdus, Plesiomonas, Pragia, Proteus, Providencia, Rahnella, Raoultella, Salmonella, Samsonia, Serratia, Shigella, Sodalis, Tatumella, Trabulsiella, Wigglesworthia, Xenorhabdus, Yersinia* oder *Yokenella,* nach Reduktasen gesucht werden, die die o.g. Modellreaktion katalysieren und deren Aminosäuresequenz die geforderte Sequenzidentität zu SEQ ID NO: 1, 2 oder 3 bereits aufweist oder durch Mutageneseverfahren erhalten wird.

Die Reduktase kann in gereinigter oder teilweise gereinigter Form oder auch in Form des Mikroorganismus selbst verwendet werden. Verfahren zur Gewinnung und Aufreinigung von Dehydrogenasen aus Mikroorganismen sind dem Fachmann hinreichend bekannt.

Bevorzugt erfolgt die die enantioselektive Reduktion mit der Reduktase in Gegenwart eines geeigneten Cofaktors (auch als Cosubstrat bezeichnet). Als Cofaktoren für die Reduktion des Ketons dient üblicherweise NADH und/oder NADPH. Daneben können Reduktasen als zelluläre Systeme eingesetzt werden, die inherent Cofaktor enthalten, oder alternative Redoxmediatoren zugesetzt werden (A. Schmidt, F. Hollmann und B. Bühler "Oxidation of Alcohols" in K. Drauz und H. Waldmann, Enzyme Catalysis in Organic Synthesis 2002, Vol.III, 991-1032, Wiley-VCH, Weinheim).

Bevorzugt erfolgt die enantioselektive Reduktion mit der Reduktase außerdem in Gegenwart eines geeigneten Reduktionsmittels, welches den im Verlauf der Reduktion oxidierten Cofaktor regeneriert. Beispiele für geeignete Reduktionsmittels sind Zucker, insbesondere Hexosen, wie Glucose, Mannose, Fructose, und/oder oxidierbare Alkohole, insbesondere Ethanol, Propanol oder Isopropanol, sowie Formiat, Phosphit oder molekularer Wasserstoff. Zur Oxidation des Reduktionsmittels und damit verbunden zur Regeneration des Coenzyms kann eine zweite Dehydrogenase, wie z.B. Glucose-dehydrogenase bei Verwendung von Glucose als Reduktionsmittel oder FormiatDehydrogenase bei der Verwendung von Formiat als Reduktionsmittel, zugesetzt werden. Diese kann als freies oder immobilisiertes Enzym oder in Form von freien oder immobilisierten Zellen eingesetzt werden. Ihre Herstellung kann sowohl separat als auch durch Coexpression in einem (rekombinanten) Reduktase-Stamm erfolgen.

Eine bevorzugte Ausführungsform des beanspruchten Verfahrens ist die Regenerierung der Cofaktoren durch ein enzymatisches System, bei dem eine zweite Dehydrogenase, besonders bevorzugt eine Glucosedehydrogenase, verwendet wird.

Weiterhin kann es zweckmäßig sein, weitere die Reduktion fördernde Zusätze, wie z. B. Metallsalze oder Chelatbildner, wie. z. B. EDTA, zu zusetzten.

Die erfindungsgemäß verwendeten Reduktasen können frei oder immobilisiert eingesetzt werden. Unter einem immobilisierten Enzym versteht man ein Enzym, das an einen inerten Träger fixiert ist. Geeignete Trägermaterialien sowie die darauf immobilisierten Enzyme sind aus der EP-A-1149849, EP-A-1 069 183 und der DE-OS 100193773 sowie aus den darin zitierten Literaturstellen bekannt. Auf die Offenbarung dieser Schriften wird diesbezüglich in vollem Umfang Bezug genommen. Zu den geeigneten Trägermaterialien gehören beispielsweise Tone, Tonmineralien, wie Kaolinit, Diatomeenerde, Perlit, Siliciumdioxid, Aluminiumoxid, Natriumcarbonat, Calciumcarbonat, Cellulosepulver, Anionenaustauschermaterialien, synthetische Polymere, wie Polystyrol, Acrylharze, Phenolformaldehydharze, Polyurethane und Polyolefine, wie Polyethylen und Polypropylen. Die Trägermaterialien werden zur Herstellung der geträgerten Enzyme üblicherweise in einer feinteiligen, partikelförmigen Form eingesetzt, wobei poröse Formen bevorzugt sind. Die Partikelgröße des Trägermaterials beträgt üblicherweise nicht mehr als 5 mm, insbesondere nicht mehr als 2 mm (Sieblinie). Analog kann bei Einsatz der Dehydrogenase als Ganzzell-Katalysator eine freie oder immobiliserte Form gewählt werden. Trägermaterialien sind z.B. Ca-Alginat, und Carrageenan. Enzyme wie auch Zellen können auch direkt mit Glutaraldehyd vernetzt werden (Crosslinking zu CLEAs). Entsprechende und weitere Immobilisierungsverfahren sind beispielsweise in J. Lalonde und A. Margolin "Immobilization of Enzymes" in K. Drauz und H. Waldmann, Enzyme Catalysis in Organic Synthesis 2002, Vol.III, 991-1032, Wiley-VCH, Weinheim beschrieben.

Die Umsetzung kann in wässrigen oder nichtwässrigen Reaktionsmedien oder in 2-Phasensystemen oder (Mikro-)Emulsionen erfolgen. Bei den wässrigen Reaktionsmedien handelt es sich vorzugsweise um gepufferte Lösungen, die in der Regel einen pH-Wert von 4 bis 8, vorzugsweise von 5 bis 8, aufweisen. Das wässrige Lösungsmittel kann neben Wasser außerdem wenigstens einen Alkohol, z.B. Ethanol oder Isopropanol oder Dimethylsulfoxid enthalten.

Unter nicht-wässrigen Reaktionsmedien werden Reaktionsmedien verstanden, die weniger als 1 Gew.-%, vorzugsweise weniger als 0,5 Gew.-% Wasser, bezogen auf das Gesamtgewicht des flüssigen Reaktionsmediums, enthalten. Insbesondere kann die Umsetzung in einem organischen Lösungsmittel durchgeführt werden.

Geeignete organische Lösungsmittel sind beispielsweise aliphatische Kohlenwasserstoffe, vorzugsweise mit 5 bis 8 Kohlenstoffatomen, wie Pentan, Cyclopentan, Hexan, Cyclohexan, Heptan, Octan oder Cyclooctan, halogenierte aliphatische Kohlenwasserstoffe, vorzugsweise mit einem oder zwei Kohlenstoffatomen, wie Dichlormethan, Chloroform, Tetrachlorkohlenstoff, Dichlorethan oder Tetrachlorethan, aromatische Kohlenwasserstoffe, wie Benzol, Toluol, die Xylole, Chlorbenzol oder Dichlorbenzol, aliphatische acyclische und cyclische Ether oder Alkohole, vorzugsweise mit 4 bis 8 Kohlenstoffatomen, wie Ethanol, Isopropanol, Diethylether, Methyl-tert-butylether, Ethyl-tert-butylether, Dipropylether, Diisopropylether, Dibutylether, Tetrahydrofuran oder Ester wie Ethylacetat oder n-Butylacetat oder Ketone wie Methylisobutylketon oder Dioxan oder Gemische davon. Besonders bevorzugt werden die vorgenannten Ether, insbesondere Tetrahydrofuran, verwendet.

Beispielsweise kann die Reduktion mit der Reduktase in einem wässrig-organischen Reaktionsmedium, wie z. B. Wasser/Isopropanol, in beliebigem Mischungsverhältnis wie z.B. 1:99 bis 99:1 oder 10: 90 bis 90:10, oder einem wässrigen Reaktionsmedium durchgeführt werden.

Das Substrat (1) wird vorzugsweise in einer Konzentration von 0,1 g/l bis 500 g/l, besonders bevorzugt von 1 g/l bis 50 g/l in die enzymatische Reduktion eingesetzt und kann kontinuierlich oder diskontinuierlich nachgeführt werden.

Die enzymatische Reduktion erfolgt in der Regel bei einer Reaktionstemperatur unterhalb der Desaktivierungstemperatur der eingesetzten Reduktase und oberhalb von -10 °C. Besonders bevorzugt liegt sie im Bereich von 0 bis 100 °C, insbesondere von 15 bis 60 °C und speziell von 20 bis 40 °C, z.B. bei etwa 30 °C.

Eine bevorzugte Ausführung des erfindungsgemäßen Verfahrens besteht darin, die Reaktion in Gegenwart von zweiwertigen Metallionen, insbesondere in Gegenwart von Ca-, Mg-, Mn-, Zn-, Ni- Fe-, Mo-Ionen durchzuführen. Vorteilhaft ist es, die Konzentration der Erdalkaliionen etwa gleich hoch wie die Konzentration des einzusetzenden Substrates (Alken-Derivat der allgemeinen Formel I) zu wählen. Insbesondere wenn das Substrat aufgrund seiner Struktur in der Lage ist, Metallionen zu komplexieren, z.B. bei Dicarbonsäurederivaten, empfiehlt sich die Zugabe von zweiwertigen Metallionen in äquimolarer Konzentration wie das Substrat.

Zur Durchführung kann man beispielsweise das Substrat (1) mit der Reduktase, dem Lösungsmittel und gegebenenfalls den Coenzymen, gegebenenfalls einer zweiten Dehydrogenase zur Regenerierung des Coenzyms und/oder weiteren Reduktionsmitteln vorlegen und das Gemisch durchmischen, z. B. durch Rühren oder Schütteln. Es ist aber auch möglich, die Reduktase in einem Reaktor, beispielsweise in einer Säule, zu immobilisieren, und durch den Reaktor eine das Substrat und gegebenenfalls Coenzyme und/oder Cosubstrate enthaltende Mischung zu leiten. Hierzu kann man die Mischung im Kreislauf durch den Reaktor leiten bis der gewünschte Umsatz erreicht ist.

In der Regel wird man die Reduktion bis zu einem Umsatz von wenigstens 70 %, besonders bevorzugt von wenigstens 85 % und insbesondere von wenigstens 95%, bezogen auf das in der Mischung enthaltene Substrat führen. Das Fortschreiten der Reaktion, d. h. die sequentielle Reduktion der Doppelbindung kann dabei durch übliche Methoden wie Gaschromatographie oder Hochdruckflüssigkeitschromatographie verfolgt werden.

"Funktionale Äquivalente" oder Analoga der konkret offenbarten Enzyme sind im Rahmen der vorliegenden Erfindung davon verschiedene Polypeptide, welche weiterhin die gewünschte biologische Aktivität, wie z.B. Substratspezifität, besitzen. So versteht man beispielsweise unter "funktionalen Äquivalenten" Enzyme, die die Modellreaktion katalysieren und die mindestens 20 %, bevorzugt 50 %, besonders bevorzugt 75 %, ganz besonders bevorzugt 90 % der Aktivität eines Enzyms, umfassend eine der unter SEQ ID NO:1, 2 oder 3 aufgeführten Aminosäuresequenzen, aufweist. Funktionale Äquivalente sind außerdem vorzugsweise zwischen pH 4 bis 10 stabil und besitzen vorteilhaft ein pH-Optimum zwischen pH 5 und 8 sowie ein Temperaturoptimum im Bereich von 20°C bis 80°C.

Unter "funktionalen Äquivalenten" versteht man erfindungsgemäß insbesondere auch Mutanten, welche in wenigstens einer Sequenzposition der oben genannten Aminosäuresequenzen eine andere als die konkret genannte Aminosäure aufweisen aber trotzdem eine der oben genannten biologischen Aktivitäten besitzen. "Funktionale Äquivalente" umfassen somit die durch eine oder mehrere Aminosäure-Additionen, - Substitutionen, -Deletionen und/oder -Inversionen erhältlichen Mutanten, wobei die genannten Veränderungen in jeglicher Sequenzposition auftreten können, solange sie zu einer Mutante mit dem erfindungsgemäßen Eigenschaftsprofil führen. Funktionale Äquivalenz ist insbesondere auch dann gegeben, wenn die Reaktivitätsmuster zwischen Mutante und unverändertem Polypeptid qualitativ übereinstimmen, d.h. beispielsweise gleiche Substrate mit unterschiedlicher Geschwindigkeit umgesetzt werden.

Beispiele für geeignete Aminosäuresubstitutionen sind folgender Tabelle zu entnehmen:

| Ursprünglicher Rest | Beispiele der Substitution |
|---|---|
| Ala | Ser |
| Arg | Lys |
| Asn | Gln; His |
| Asp | Glu |
| Cys | Ser |
| Gln | Asn |
| Glu | Asp |
| Gly | Pro |
| His | Asn ; Gln |
| Ile | Leu; Val |
| Leu | Ile; Val |
| Lys | Arg ; Gln ; Glu |
| Met | Leu ; Ile |
| Phe | Met ; Leu ; Tyr |
| Ser | Thr |
| Thr | Ser |
| Trp | Tyr |
| Tyr | Trp ; Phe |
| Val | Ile; Leu |

"Funktionale Äquivalente" im obigen Sinne sind auch "Präkursoren" der beschriebenen Polypeptide sowie "funktionale Derivate".

"Präkursoren" sind dabei natürliche oder synthetische Vorstufen der Polypeptide mit oder ohne der gewünschten biologischen Aktiviät.

"Funktionale Derivate" erfindungsgemäßer Polypeptide können an funktionellen Aminosäure-Seitengruppen oder an deren N- oder C-terminalen Ende mit Hilfe bekannter Techniken ebenfalls hergestellt werden. Derartige Derivate umfassen beispielsweise aliphatische Ester von Carbonsäuregruppen, Amide von Carbonsäuregruppen, erhältlich durch Umsetzung mit Ammoniak oder mit einem primären oder sekundären Amin; N-Acylderivate freier Aminogruppen, hergestellt durch Umsetzung mit Acylgruppen; oder O-Acylderivate freier Hydroxygruppen, hergestellt durch Umsetzung mit Acylgruppen.

Im Falle einer möglichen Proteinglykosylierung umfassen erfindungsgemäße "funktionale Äquivalente" Proteine des oben bezeichneten Typs in deglykosylierter bzw. glykosylierter Form sowie durch Veränderung des Glykosylierungsmusters erhältliche abgewandelte Formen.

"Funktionale Äquivalente" umfassen natürlich auch Polypeptide welche aus anderen Organismen zugänglich sind, sowie natürlich vorkommende Varianten. Beispielsweise lassen sich durch Sequenzvergleich Bereiche homologer Sequenzregionen festlegen und in Anlehnung an die konkreten Vorgaben der Erfindung äquivalente Enzyme ermitteln.

"Funktionale Äquivalente" umfassen ebenfalls Fragmente, vorzugsweise einzelne Domänen oder Sequenzmotive, der erfindungsgemäßen Polypeptide, welche z.B. die gewünschte biologische Funktion aufweisen.

"Funktionale Äquivalente" sind außerdem Fusionsproteine, welche eine der oben genannten Polypeptidsequenzen oder davon abgeleitete funktionale Äquivalente und wenigstens eine weitere, davon funktionell verschiedene, heterologe Sequenz in funktioneller N- oder C-terminaler Verknüpfung (d.h. ohne gegenseitigen wesentliche funktionelle Beeinträchtigung der Fusionsproteinteile) aufweisen. Nichtlimitierende Beispiele für derartige heterologe Sequenzen sind z.B. Signalpeptide oder Enzyme.

Homologe des erfindungsgemäßen Proteine können durch Screening kombinatorischer Banken von Mutanten, wie z.B. Verkürzungsmutanten, identifiziert werden. Beispielsweise kann eine variegierte Bank von Protein-Varianten durch kombinatorische Mutagenese auf Nukleinsäureebene erzeugt werden, wie z.B. durch enzymatisches Ligieren eines Gemisches synthetischer Oligonukleotide. Es gibt eine Vielzahl von Verfahren, die zur Herstellung von Banken potentieller Homologer aus einer degenerierten Oligonukleotidsequenz verwendet werden können. Die chemische Synthese einer degenerierten Gensequenz kann in einem DNA-Syntheseautomaten durchgeführt werden, und das synthetische Gen kann dann in einen geeigneten Expressionsvektor ligiert werden. Die Verwendung eines degenerierten Gensatzes ermöglicht die Bereitstellung sämtlicher Sequenzen in einem Gemisch, die den gewünschten Satz an potentiellen Proteinsequenzen kodieren. Verfahren zur Synthese degenerierter Oligonukleotide sind dem Fachmann bekannt (z.B. Narang, S.A. (1983) Tetrahedron 39:3; Itakura et al. (1984) Annu. Rev. Biochem. 53:323; Itakura et al., (1984) Science 198:1056; Ike et al. (1983) Nucleic Acids Res. 11:477).

Im Stand der Technik sind mehrere Techniken zum Screening von Genprodukten kombinatorischer Banken, die durch Punktmutationen oder Verkürzung hergestellt worden sind, und zum Screening von cDNA-Banken auf Genprodukte mit einer ausgewählten Eigenschaft bekannt. Diese Techniken lassen sich an das schnelle Screening der Genbanken anpassen, die durch kombinatorische Mutagenese erfindungsgemäßer Homologer erzeugt worden sind. Die am häufigsten verwendeten Techniken zum Screening großer Genbanken, die einer Analyse mit hohem Durchsatz unterliegen, umfassen das Klonieren der Genbank in replizierbare Expressionsvektoren, Transformieren der geeigneten Zellen mit der resultierenden Vektorenbank und Exprimieren der kombinatorischen Gene unter Bedingungen, unter denen der Nachweis der gewünschten Aktivität die Isolation des Vektors, der das Gen kodiert, dessen Produkt nachgewiesen wurde, erleichtert. Recursive-Ensemble-Mutagenese (REM), eine Technik, die die Häufigkeit funktioneller Mutanten in den Banken vergrößert, kann in Kombination mit den Screeningtests verwendet werden, um Homologe zu identifizieren (Arkin und Yourvan (1992) PNAS 89:7811-7815; Delgrave et al. (1993) Protein Engineering 6(3):327-331).

Gegenstand der Erfindung sind weiterhin Nukleinsäuresequenzen (einzel- und doppelsträngige DNA- und RNA-Sequenzen, wie z.B. cDNA und mRNA), die für ein Enzym mit erfindungsgemäßer Reduktase -Aktivität kodieren. Bevorzugt sind Nukleinsäuresequenzen, welche z.B. für Aminosäuresequenzen gemäß SEQ ID NO:1, 2 oder 3 charakteristische Teilsequenzen davon kodieren.

Alle hierin erwähnten Nukleinsäuresequenzen sind in an sich bekannter Weise durch chemische Synthese aus den Nukleotidbausteinen, wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, Seiten 896-897) erfolgen. Die Anlagerung synthetischer Oligonukleotide und Auffüllen von Lücken mit Hilfe des Klenow-Fragmentes der DNA-Polymerase und Ligationsreaktionen sowie allgemeine Klonierungsverfahren werden in Sambrook et al. (1989), Molecular Cloning: A laboratory manual, Cold Spring Harbor Laboratory Press, beschrieben.

Weitere Ausgestaltungen zur Durchführung des erfindungsgemäßen enzymatischen Reduktionsverfahrens:

Der pH-Wert im erfindungsgemäßen Verfahren wird vorteilhaft zwischen pH 4 und 12, bevorzugt zwischen pH 4,5 und 9, besonders bevorzugt zwischen pH 5 und 8 gehalten. min. 98 %ee erreicht.

Für das erfindungsgemäße Verfahren können wachsende Zellen verwendet werden, die für die Reduktase kodierenden Nukleinsäuren, Nukleinsäurekonstrukte oder Vektoren enthalten. Auch ruhende oder aufgeschlossene Zellen können verwendet werden. Unter aufgeschlossenen Zellen sind beispielsweise Zellen zu verstehen, die über eine Behandlung mit beispielsweise Lösungsmitteln durchlässig gemacht worden sind, oder Zellen die über eine Enzymbehandlung, über eine mechanische Behandlung (z.B. French Press oder Ultraschall) oder über eine sonstige Methode aufgebrochen wurden. Die so erhaltenen Rohextrakte sind für das erfindungsgemäße Verfahren vorteilhaft geeignet. Auch gereinigte oder angereinigte Enzyme können für das Verfahren verwendet werden. Ebenfalls geeignet sind immobilisierte Mikroorganismen oder Enzyme, die vorteilhaft in der Reaktion Anwendung finden können.

Das erfindungsgemäße Verfahren kann batchweise, semi-batchweise oder kontinuierlich betrieben werden.

Die Durchführung des Verfahrens kann vorteilhafterweise in Bioreaktoren erfolgen, wie z.B. beschrieben in Biotechnology, Band 3, 2. Auflage, Rehm et al Hrsg., (1993) insbesondere Kapitel II.

Die in dem erfindungsgemäßen Verfahren hergestellten Produkte können aus dem Reaktionsmedium mit dem Fachmann geläufigen Verfahren isoliert und gewünschtenfalls aufgereinigt werden. Hierzu zählen Destillationsverfahren, chromatographische Verfahren, Extraktionsverfahren und Kristallisationsverfahren. Die Reinigung der Produkte kann je nach Anforderung durch Kombination mehrerer dieser Verfahren deutlich erhöht werden.

Die nachfolgenden Beispiele sollen die Erfindung veranschaulichen, ohne sie jedoch einzuschränken. Hierbei wird auf beiliegende Abbildungen Bezug genommen, dabei zeigt:

### Experimenteller Teil

### Allgemeine Vorschrift zur asymmetrischen Bioreduktion

Die asymmetrische Bioreduktion der Substrate wurde gemäß der folgenden allgemeinen Vorschrift unter Verwendung der isolierten Enzyme YqjM, OPR1, OPR3 durchgeführt. Wegen der schlechten Wasserlöslichkeit wurde N-Phenyl-2-methylmaleimid als 10% DMF Lösung zugesetzt (1% Endkonzentration).

Die Enzympräparation (100-200µg) wurde zu einer Lösung des Substrats (5mM) in Tris Puffer 50 mM ph /,5 (0,8ml) mit dem Cofaktor NADH oder NADPH (15 mM) zugesetzt und die Reaktion wurde bei 30°C unter Schütteln (140rpm) durchgeführt. Nach 48 Stunden wurde das Reaktionsgemisch mit Ethylacetat extrahiert und die Reaktionsprodukte über GC analysiert.

Bei Verwendung des Cofaktor-Recycling-Systems, wurde folgende Vorgehensweise gewählt:
NADH/FDH System
   Zu einer Mischung aus Substrat (5mM) oxidiertem Cofaktor NAD⁺ (100µM), Ammoniumfomat (20mM) in Tris Puffer 50mM pH 7,5 (0,8ml) wurde FDH (10u) zugegeben nachdem das Enzym (100-200µg) zugegeben worden war und die Reaktion wurde bei 30°C (140 rpm) für 48 Stunden durchgeführt.
NADH/GDH
   Zu einer Mischung aus Substrat (5mM) oxidiertem Cofaktor NAD⁺ (100µM), Glucose (20mM) in Tris Puffer 50mM pH 7,5 (0,8ml) wurde (D)-GDH (10u) zugegeben nachdem das Enzym (100-200µg) zugegeben worden war und die Reaktion wurde bei 30°C (140 rpm) für 48 Stunden durchgeführt.
NADPH/G6PDH
   Zu einer Mischung aus Substrat (5mM) oxidiertem Cofaktor NADP⁺ (10µM), Glucose-6-Phosphat (20mM) in Tris Puffer 50mM pH 7,5 (0,8ml) wurde G6PDH (10u) zugegeben nachdem das Enzym (100-200µg) zugegeben worden war und die Reaktion wurde bei 30°C (140 rpm) für 48 Stunden durchgeführt.
GC-FID Analysen wurden auf einem Varian 3800 Gas Chromatographen mit H₂ als Carriergas (14,5psi) durchgeführt.
1-Nitro-2-phenylpropen:
   Bestimmung der Umsetzung: Die erhaltenen Produkte wurden durch GC-FID analysiert unter Verwendung einer 6% Cyanopropylphenylpolysiloxane Phasenkapillarsäule (Varian CP-1301, 30m, 0,25 mm, 0,25µm film) mit einem split-ratio von 30:1. Programm: 120°C/min bis 180°C, 20°C/min bis 220°C, 2 Min. Halt. Retentionszeiten waren folgende: Limonen (interner Standard) 3,81 min, 1-nitro-2-phenylpropan 8,87 Min, 1-Nitro-2-phenylproen Z/E 9,55/10,27 min resp.
   Bestimmung des enationmeren Überschuss und der absoluten Konfiguration: Der Enantiomerenüberschuss wurde unter Verwendung einer Cyclodextrin-gebundenen Dimethylpolysiloxan Phasenkapillarsäule (CP-Chirasil-DEX CB, 25 m, 0,32 mm, 0,25 µm Film) mit einem split-ratio von 25:1. Temperaturprogramm: 105°C 5 min Halt, 1°C/min bis 120°C, 6 min. Halt, 20°C/min bis 180°C, 2 Min. Halt. Retentionszeiten waren folgende: (S)- und (R)-1-Nitro-2-phenylpropan 12,06 und 12,57 min, resp. Die absolute Konfiguration von 1-Nitro-2-phenylpropan wurde durch Coinjection einer unabhängig synthetisierten Referenzprobe (J. Org. Chem. 1989, 54, 1802-1804) ermittelt.
Citral
   Bestimmung der Umsetzung: Die erhaltenen Produkte wurden durch GC-FID analysiert unter Verwendung einer Polyethylenglykol Phasenkapillarsäule (Varian CP-Wax 52CB, 30m, 0,25 mm, 0,25µm film) mit einem split-ratio von 20:1. Programm: 100°C Halt für 2 min., 15°C/min bis 240°C, 10 Min. Halt. Retentionszeiten waren folgende: Citronellal 5,21 min, 1-Octanol (interner Standard) 5,83 min, Geranial 7,53 min
   Bestimmung des enationmeren Überschuss und der absoluten Konfiguration: Der Enantiomerenüberschuss von Citronellal wurde unter Verwendung einer modifizierten ß-Cyclodextrin Kapillarsäule (Hydrodex-ß-TBDAc, 25 m, 0,25 mm) Temperaturprogramm: 40°C Halt 2 min, 4°C/min bis 120°C, 1 min. Halt, 20°C/min bis 180°C, 3 Min. Halt. Retentionszeiten waren folgende: (S)- und (R)-Citronellal 19,84und 19,97 min resp. Die absolute Konfiguration von Citronellal wurde durch Coinjection einer kommerziell erhältlichen Referenzprobe mit bekannter absoluter Konfiguration ermittelt.
N-Pheny-2-methylmaleimid:
   Bestimmung der Umsetzung: Die erhaltenen Produkte wurden durch GC-FID analysiert unter Verwendung einer 6% Cyanopropylphenylpolysiloxane Phasenkapillarsäule (Varian CP-1301, 30m, 0,25 mm, 0,25µm film) mit einem split-ratio von 30:1. Programm: 110°C Halt 2 min, 30°C/min bis 210°C, 6 Min. Halt. Retentionszeiten waren folgende: Limonen (interner Standard) 3,69 min, N-Phenyl-2-methylmaleimid 8,77min, N-phenyl-2-methyl-succinimid 9,89 min.
   Bestimmung des enationmeren Überschuss und der absoluten Konfiguration: Der Enantiomerenüberschuss wurde an einer Shimadzu Chiral HPLC unter Verwendung einer Chiralcel OD-H Säule und einer Lösung aus n-Heptan/Ethanol 95:5 als Eluent bestimmt. 80µl wurden isokratisch bei 18°C über 33 min eluiert. Retentionszeiten waren folgende: (S)- und (R)-N-Phenyl-2-methylsuccinimid 27,0 und 29,1 min resp. Die absolute Konfiguration von 1-N-Phenyl-2-methylsuccinimid wurde durch CD Spektroskopie (J. Mol. Catal. B:Enzym 2005, 32, 131-134) ermittelt.
Cyclische Enone:
   Bestimmung der Umsetzung: Die erhaltenen Produkte wurden durch GC-FID analysiert unter Verwendung einer 6% Cyanopropylphenylpolysiloxane Phasenkapillarsäule (Varian CP-1301, 30m, 0,25 mm, 0,25µm film) mit einem split-ratio von 30:1. Programm:
   80°C Halt für 10 min, 30°C/min bis 200°C, 3 Min. Halt. Retentionszeiten waren folgende: 2-Methylcyclopentanon 4,27 min, 3-Methylcyclopentanon 4,44 min, 2-Methyl-2-cyclo-penten-1-on 5,82min,3-Methylcylclohexanon 7,27 min, (R)-Limonen (interner Standard) 8,59 min, 3-Methyl-2-cyclopenten-1on 8,77 min, 3-Methyl-2-cyclohexen-1-on 11,77 min. resp.
   Bestimmung des enationmeren Überschuss und der absoluten Konfiguration: Der Enantiomerenüberschuss wurd unter Verwendung einer modifizierten ß-Cyclodextrin-Kapillarsäule (Chiraldex B-TA, 40 m, 0,25 mm) mit einem split-ratio von 25:1. Temperaturprogramm: 80°C 17 min Halt, 30°C/min bis 180°C, 2 Min. Halt. Retentionszeiten waren folgende: (S)- und (R)-3-Methylcyclopentanon 8,08 und 8,29 min, (R)- und (S)-2-Methylcyclopentanon 6,55 und 6,77 min, (R)- und (S)-3-Methylcyclohexanon 13,96 und 15,09 min. Die absolute Konfiguration von 3-Methylcylcopentanon und 3-Methylcyclohexanon wurde durch Coinjection einer kommerziell erhältlichen Referenzprobe mit bekannter absoluter Konfiguration ermittelt.. Die absolute Konfiguration von 2-Methylcylcopentanon wurde durch Coinjection einer unabhängig synthetisierten Referenzprobe mit bekannter absoluter Konfiguration ermittelt (Tetrahedron:Asymmety 2001, 12, 1479-1483).

## Patentansprüche

1. Verfahren zur enzymatischen Herstellung von Verbindungen der allgemeinen Formel (2) aus ungesättigten Alken-Derivaten der allgemeinen Formel (1) 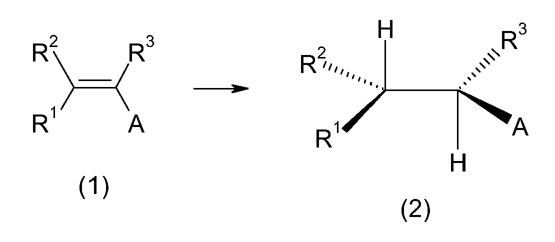 a
worin
A für einen Iminrest (=NR), einen Nitrorest (-NO₂), einen Ketonrest (-CRO), einen Aldehydrest (-CHO), einen Carboxylrest (-COOR) mit R= H oder gegebenenfalls substituerter C₁-C₆- Alkylrest, steht,
R¹, R² und R³ unabhängig voneinander für H, C₁-C₆- Alkyl, C₂-C₆- Alkenyl, Carboxyl oder einen gegebenenfalls substituierten carbo- oder heterocyclischen, aromatischen oder nichtaromatischen Rest steht, oder R¹ ist mit R³ so verknüpt, dass sie Teil eines 4-8 gliedrigen Cyclus werden, oder R¹ ist mit R so verknüpt, dass sie Teil eines 4-8 gliedrigen Cyclus werden,
durch Reduktion einer Verbindung der Formel (1) in Gegenwart einer Reduktase
(i) umfassend wenigstens eine der Polypeptidsequenzen SEQ ID NO: 1, 2, oder 3 oder
(ii) mit einer funktional äquivalenten Polypeptidsequenz, die mindestens 80% Sequenzidentität mit SEQ ID NO:1, 2, oder 3 aufweist.

2. Verfahren nach Anspruch 1, **dadurch gekennzeichnet, dass** die Reduktion mit NADPH oder NADH als Cofaktor durchgeführt wird.

3. Verfahren nach Anspruch 2, **dadurch gekennzeichnet, dass** der verwendete Cofaktor enzymatisch regeneriert wird.

4. Verfahren nach Anspruch 3, **dadurch gekennzeichnet, dass** die Cofaktor-Regenerierung durch Glucose-Dehydrogenase oder Formiatdehydrogenase oder einen sekundären Alkohol erfolgt.

5. Verfahren nach einem der vorhergehenden Ansprüche, **dadurch gekennzeichnet, dass** die Reduktion in einem wässrigen, wässrig-alkoholischen oder alkoholischen Reaktionsmedium erfolgt.

6. Verfahren nach einem der vorhergehenden Ansprüche, **dadurch gekennzeichnet, dass** die Reduktase immobilisiert vorliegt.

7. Verfahren nach einem der vorhergehenden Ansprüche, wobei das Enzym ausgewählt ist unter Reduktasen aus Bacillus subtilis und Lycopersicum esculentum.

8. Verfahren nach einem der vorhergehenden Ansprüche, wobei eine Verbindung der Formel (1) umgesetzt wird, worin R¹ für Methyl und A für einen Ketonrest steht.

9. Verfahren nach einem der vorhergehenden Ansprüche, wobei die Reaktion bei einer Temperatur im Bereich von 0 bis 45°C und/oder einem pH-Wert im Bereich von 6 bis 8 erfolgt.

10. Verfahren nach einem der vorhergehenden Ansprüche, wobei die Reaktion in-Gegenwart von zweiwertigen Metallionen durchgeführt wird.

11. Verwendung einer Verbindung der Formel (2), hergestellt nach einem Verfahren nach einem der vorhergehenden Ansprüche als Zwischenprodukt für die chemische oder enzymatische Wirkstoffsynthese.


SEQUENCE LISTING

<110>  BASF Aktiengesellschaft

<120>  Verfahren zur enzymatischen Reduktion von Alkenderivaten

<130>  PF 58579-2

<160>  3

<170>  PatentIn version 3.1

<210>  1
<211>  337
<212>  PRT
<213>  Bacillus subtilis

<400>  1

Ala Arg Lys Leu Phe Thr Pro Ile Thr Ile Lys Asp Met Thr Leu Lys
1               5                   10                  15

Asn Arg Ile Val Met Ser Pro Met Cys Met Tyr Ser Ser His Glu Lys
            20                  25                  30

Asp Gly Lys Leu Thr Pro Phe His Met Ala His Tyr Ile Ser Arg Ala
        35                  40                  45

Ile Gly Gln Val Gly Leu Ile Ile Val Glu Ala Ser Ala Val Asn Pro
    50                  55                  60

Gln Gly Arg Ile Thr Asp Gln Asp Leu Gly Ile Trp Ser Asp Glu His
65                  70                  75                  80

Ile Glu Gly Phe Ala Lys Leu Thr Glu Gln Val Lys Glu Gln Gly Ser
                85                  90                  95

Lys Ile Gly Ile Gln Leu Ala His Ala Gly Arg Lys Ala Glu Leu Glu
            100                 105                 110

Gly Asp Ile Phe Ala Pro Ser Ala Ile Ala Phe Asp Glu Gln Ser Ala
        115                 120                 125

Thr Pro Val Glu Met Ser Ala Glu Lys Val Lys Glu Thr Val Gln Glu

130                 135                 140

Phe Lys Gln Ala Ala Ala Arg Ala Lys Glu Ala Gly Phe Asp Val Ile
145                 150                 155                 160

Glu Ile His Ala Ala His Gly Tyr Leu Ile His Glu Phe Leu Ser Pro
                165                 170                 175

Leu Ser Asn His Arg Thr Asp Glu Tyr Gly Gly Ser Pro Glu Asn Arg
            180                 185                 190

Tyr Arg Phe Leu Arg Glu Ile Ile Asp Glu Val Lys Gln Val Trp Asp
        195                 200                 205

Gly Pro Leu Phe Val Arg Val Ser Ala Ser Asp Tyr Thr Asp Lys Gly
    210                 215                 220

Leu Asp Ile Ala Asp His Ile Gly Phe Ala Lys Trp Met Lys Glu Gln
225                 230                 235                 240

Gly Val Asp Leu Ile Asp Cys Ser Ser Gly Ala Leu Val His Ala Asp
                245                 250                 255

Ile Asn Val Phe Pro Gly Tyr Gln Val Ser Phe Ala Glu Lys Ile Arg
            260                 265                 270

Glu Gln Ala Asp Met Ala Thr Gly Ala Val Gly Met Ile Thr Asp Gly
        275                 280                 285

Ser Met Ala Glu Glu Ile Leu Gln Asn Gly Arg Ala Asp Leu Ile Phe
    290                 295                 300

Ile Gly Arg Glu Leu Leu Arg Asp Pro Phe Phe Ala Arg Thr Ala Ala
305                 310                 315                 320

Lys Gln Leu Asn Thr Glu Ile Pro Ala Pro Val Gln Tyr Glu Arg Gly
                325                 330                 335

Trp

<210>  2

<211>  376

<212>  PRT

<213>  Lycopersicon esculentum

<400>  2

Met Glu Asn Lys Val Val Glu Glu Lys Gln Val Asp Lys Ile Pro Leu
1               5                   10                  15

Met Ser Pro Cys Lys Met Gly Lys Phe Glu Leu Cys His Arg Val Val
            20                  25                  30

Leu Ala Pro Leu Thr Arg Gln Arg Ser Tyr Gly Tyr Ile Pro Gln Pro
        35                  40                  45

His Ala Ile Leu His Tyr Ser Gln Arg Ser Thr Asn Gly Gly Leu Leu
    50                  55                  60

Ile Gly Glu Ala Thr Val Ile Ser Glu Thr Gly Ile Gly Tyr Lys Asp
65                  70                  75                  80

Val Pro Gly Ile Trp Thr Lys Glu Gln Val Glu Ala Trp Lys Pro Ile
                85                  90                  95

Val Asp Ala Val His Ala Lys Gly Gly Ile Phe Phe Cys Gln Ile Trp
            100                 105                 110

His Val Gly Arg Val Ser Asn Lys Asp Phe Gln Pro Asn Gly Glu Asp
        115                 120                 125

Pro Ile Ser Cys Thr Asp Arg Gly Leu Thr Pro Gln Ile Arg Ser Asn
    130                 135                 140

Gly Ile Asp Ile Ala His Phe Thr Arg Pro Arg Arg Leu Thr Thr Asp
145                 150                 155                 160

Glu Ile Pro Gln Ile Val Asn Glu Phe Arg Val Ala Ala Arg Asn Ala
                165                 170                 175

Ile Glu Ala Gly Phe Asp Gly Val Glu Ile His Gly Ala His Gly Tyr
            180                 185                 190

Leu Ile Asp Gln Phe Met Lys Asp Gln Val Asn Asp Arg Ser Asp Lys
        195                 200                 205

Tyr Gly Gly Ser Leu Glu Asn Arg Cys Arg Phe Ala Leu Glu Ile Val
    210                 215                 220

Glu Ala Val Ala Asn Glu Ile Gly Ser Asp Arg Val Gly Ile Arg Ile
225                 230                 235                 240

Ser Pro Phe Ala His Tyr Asn Glu Ala Gly Asp Thr Asn Pro Thr Ala
                245                 250                 255

Leu Gly Leu Tyr Met Val Glu Ser Leu Asn Lys Tyr Asp Leu Ala Tyr
            260                 265                 270

Cys His Val Val Glu Pro Arg Met Lys Thr Ala Trp Glu Lys Ile Glu
        275                 280                 285

Cys Thr Glu Ser Leu Val Pro Met Arg Lys Ala Tyr Lys Gly Thr Phe
    290                 295                 300

Ile Val Ala Gly Gly Tyr Asp Arg Glu Asp Gly Asn Arg Ala Leu Ile
305                 310                 315                 320

Glu Asp Arg Ala Asp Leu Val Ala Tyr Gly Arg Leu Phe Ile Ser Asn
                325                 330                 335

Pro Asp Leu Pro Lys Arg Phe Glu Leu Asn Ala Pro Leu Asn Lys Tyr
            340                 345                 350

Asn Arg Asp Thr Phe Tyr Thr Ser Asp Pro Ile Val Gly Tyr Thr Asp
        355                 360                 365

Tyr Pro Phe Leu Glu Thr Met Thr
    370                 375

<210>  3

<211>  396

<212>  PRT

<213>  Lycopersicon esculentum


<400>  3

Met Ala Ser Ser Ala Gln Asp Gly Asn Asn Pro Leu Phe Ser Pro Tyr
1               5                   10                  15

Lys Met Gly Lys Phe Asn Leu Ser His Arg Val Val Leu Ala Pro Met
            20                  25                  30

Thr Arg Cys Arg Ala Leu Asn Asn Ile Pro Gln Ala Ala Leu Gly Glu
        35                  40                  45

Tyr Tyr Glu Gln Arg Ala Thr Ala Gly Gly Phe Leu Ile Thr Glu Gly
    50                  55                  60

Thr Met Ile Ser Pro Thr Ser Ala Gly Phe Pro His Val Pro Gly Ile
65                  70                  75                  80

Phe Thr Lys Glu Gln Val Arg Glu Trp Lys Lys Ile Val Asp Val Val
                85                  90                  95

His Ala Lys Gly Ala Val Ile Phe Cys Gln Leu Trp His Val Gly Arg
            100                 105                 110

Ala Ser His Glu Val Tyr Gln Pro Ala Gly Ala Ala Pro Ile Ser Ser
        115                 120                 125

Thr Glu Lys Pro Ile Ser Asn Arg Trp Arg Ile Leu Met Pro Asp Gly
    130                 135                 140

Thr His Gly Ile Tyr Pro Lys Pro Arg Ala Ile Gly Thr Tyr Glu Ile
145                 150                 155                 160

Ser Gln Val Val Glu Asp Tyr Arg Arg Ser Ala Leu Asn Ala Ile Glu
                165                 170                 175

Ala Gly Phe Asp Gly Ile Glu Ile His Gly Ala His Gly Tyr Leu Ile
            180                 185                 190

Asp Gln Phe Leu Lys Asp Gly Ile Asn Asp Arg Thr Asp Glu Tyr Gly
        195                 200                 205

Gly Ser Leu Ala Asn Arg Cys Lys Phe Ile Thr Gln Val Val Gln Ala
    210                 215                 220

Val Val Ser Ala Ile Gly Ala Asp Arg Val Gly Val Arg Val Ser Pro
225                 230                 235                 240

Ala Ile Asp His Leu Asp Ala Met Asp Ser Asn Pro Leu Ser Leu Gly
                245                 250                 255

Leu Ala Val Val Glu Arg Leu Asn Lys Ile Gln Leu His Ser Gly Ser
            260                 265                 270

Lys Leu Ala Tyr Leu His Val Thr Gln Pro Arg Tyr Val Ala Tyr Gly
        275                 280                 285

Gln Thr Glu Ala Gly Arg Leu Gly Ser Glu Glu Glu Glu Ala Arg Leu
    290                 295                 300

Met Arg Thr Leu Arg Asn Ala Tyr Gln Gly Thr Phe Ile Cys Ser Gly
305                 310                 315                 320

Gly Tyr Thr Arg Glu Leu Gly Ile Glu Ala Val Ala Gln Gly Asp Ala
                325                 330                 335

Asp Leu Val Ser Tyr Gly Arg Leu Phe Ile Ser Asn Pro Asp Leu Val
            340                 345                 350

Met Arg Ile Lys Leu Asn Ala Pro Leu Asn Lys Tyr Asn Arg Lys Thr
        355                 360                 365

Phe Tyr Thr Gln Asp Pro Val Val Gly Tyr Thr Asp Tyr Pro Phe Leu
    370                 375                 380

Gln Gly Asn Gly Ser Asn Gly Pro Leu Ser Arg Leu
385                 390                 395